# EUROPÄISCHE PATENTSCHRIFT

(11) **EP 2 283 026 B1**
(45) Veröffentlichungstag und Bekanntmachung des Hinweises auf die Patenterteilung: **01.11.2017**
(21) Anmeldenummer: 09742154.9
(22) Anmeldetag: 08.05.2009
(51) Int. Cl.: C07K 1/32, C08B 37/00, C12N 7/00, C12N 15/10

(54) **VERFAHREN ZUR ANREICHERUNG UND ISOLIERUNG VON BIOMOLEKÜLEN ODER VIREN**
METHOD FOR CONCENTRATING AND ISOLATING BIOMOLECULES OR VIRUSES
PROCÉDÉ PERMETTANT D ENRICHIR ET D ISOLER DES BIOMOLÉCULES OU DES VIRUS

(30) Priorität: 08.05.2008 DE 102008023297
(43) Veröffentlichungstag der Anmeldung: 16.02.2011
(73) Patentinhaber: AJ Innuscreen GmbH, 13125 Berlin (DE)
(72) Erfinder: HILLEBRAND, Timo, 15366 Hoppegarten (DE)
(74) Vertreter: Wehlan, Helmut
(86) Internationale Anmeldenummer: PCT/EP2009/055596
(87) Internationale Veröffentlichungsnummer: WO 2009/135936

(56) Entgegenhaltungen:
- EP-A- 1 739 094
- WO-A-03/095079
- US-A- 5 739 019
- JAIN SULAKSHANA ET AL: "Applications of alginate in bioseparation of proteins." ARTIFICIAL CELLS, BLOOD SUBSTITUTES, AND IMMOBILIZATION BIOTECHNOLOGY 2006, Bd. 34, Nr. 2, 2006, Seiten 127-144, XP009121281 ISSN: 1073-1199
- TEOTIA S ET AL: "One-step purification of glucoamylase by affinity precipitation with alginate." JOURNAL OF MOLECULAR RECOGNITION : JMR 2001 SEP-OCT, Bd. 14, Nr. 5, September 2001 (2001-09), Seiten 295-299, XP002541150 ISSN: 0952-3499
- DATABASE MEDLINE [Online] US NATIONAL LIBRARY OF MEDICINE (NLM), BETHESDA, MD, US; 2004, DE CEUNINCK FRÉDÉRIC ET AL: "Culture of chondrocytes in alginate beads." XP002541151 Database accession no. NLM15280584 & METHODS IN MOLECULAR MEDICINE 2004, Bd. 100, 2004, Seiten 15-22, ISSN: 1543-1894
- MITTAL S K ET AL: "Immunization with DNA, adenovirus or both in biodegradable alginate microspheres: effect of route of inoculation on immune response", VACCINE, ELSEVIER LTD, GB, vol. 19, no. 2-3, 15 September 2000 (2000-09-15), pages 253-263, XP027321944, ISSN: 0264-410X [retrieved on 2000-09-15]

## Beschreibung

Gegenstand der Erfindung ist ein einfaches Verfahren zur Anreicherung von Nukleinsäuren und/oder Viren aus einer Probe für die nachfolgende Isolierung der Nukleinsäuren und ggf. sensitive Detektion.

Die Untersuchung diagnostisch relevanter biologischer Proben wie Serum, Plasma, Blut, Tupferproben oder Organabrieben zum Nachweis infektiöser Erreger hat in den letzten Jahren enorm an Bedeutung gewonnen. Virusinfektionen wie HIV, HCV oder HBV sind weltweit auf dem Vormarsch.

Kommerziell verfügbare Nachweisverfahren viraler Nukleinsäuren beruhen auf bekannten Nukleinsäure-Amplifikationstechniken wie PCR (Polymerase Chain Reaction), Realtime-PCR, NASBA (Nucleic Acid Sequence-Based Amplification) oder branched- DNA-Detektion. Allerdings stoßen die bekannten Nachweisverfahren insbesondere bei der Testung von großvolumigen Proben (z.B. Pools von 96 Plasma-Einzelproben) an die Grenzen der Nachweisempfindlichkeit. Die Problematik liegt darin begründet, dass die eingesetzten kommerziellen Tests immer eine Isolierung der (z.B. viralen) Nukleinsäuren bedürfen. Die Isolierung viraler Nukleinsäuren erfolgt dabei in der Regel aus flüssigen Proben mit einem Volumen von 50 - 200 µl.

Um eine höhere Nachweissensitivität erreichen zu können bzw. um auch gepoolte Einzelproben sensitiv untersuchen zu können, bedarf es Verfahren der Aufarbeitung von großvolumigen Proben. Da dies mit den gängigen Nukleinsäureisolierungsverfahren nicht möglich ist, wurde versucht, in einem ersten Schritt Viruspartikel einer Probe anzureichern. Eine Möglichkeit der Anreicherung viraler Partikel besteht in der Ultrazentrifugation der zu untersuchenden Probe. Dabei kommt es zur Ansammlung von Viruspartikeln am Boden des Reaktionsgefäßes. Diese Methode ist an eine Ultrazentrifuge gebunden und darüber hinaus zeitaufwendig und für die Routinediagnostik ungeeignet. Alternative Verfahren bestehen in der Präzipitation von Viruspartikeln mittels Polyethylenglycol/ Natriumchlorid und einer nachfolgenden Zentrifugation (Yamamoto et al., Virology 40(1970) 734; Morandi et al., J.Clin.Microbiol. 36 (1998) 1543-1538). Dabei nutzt man verschiedene Mischungen von PEG und Natriumchlorid und mischt diese Reagenzien mit der biologischen Probe. Nachfolgend wird der Ansatz bei Kälte längere Zeit inkubiert und nachfolgend die Virus(Protein)- NaCl/ PEG- Präzipitate durch Zentrifugation gewonnen. Auch diese Verfahren sind aufwendig und benötigen viel Zeit. Problematisch ist darüber hinaus die Weiterbearbeitung der Präzipitate für die Isolierung der viralen Nukleinsäuren. Oftmals lassen sich die Präzipitate nur sehr schwer wieder in Lösung bringen. Dies beeinflusst die Effizienz und die Qualität der Nukleinsäureisolierung erheblich. Die Patentschrift DE 19856415 C2 beschreibt ein Verfahren, welches sich der bekannten NaCl/ PEG- Präzipitation bedient, wobei nachfolgend die Isolierung der Nukleinsäuren in an sich bekannter Weise über die Bindung an eine silikatische feste Phase realisiert wird. Inwieweit sich dieses Verfahren von der hinlänglich bekannten Methode der NaCl/ PEG- Präzipitation mit den bekannten Problemen abhebt, wird nicht ersichtlich. Darüber hinaus bedarf auch dieses Verfahren einer Kälteinkubation und einer zwanzigminütigen Zentrifugation.

Das Verfahren soll es gestatten, virale Nukleinsäuren aus einer Probe von bis zu 10 ml zu isolieren. Eine weitere kommerziell verfügbare Variante, welche die Bearbeitung von bis zu 1 ml Probe erlauben soll, basiert auf der Anreicherung der viralen Nukleinsäuren unter Verwendung eines speziellen Detergenzes. Eine initiale Inkubation der Probe mit einem Lysereagenz führt dabei zur Lyse der Viren. Nachfolgend kommt es zur Ausbildung eines "Detergenz- Nukleinsäurekomplexes" . Der Ansatz wird zentrifugiert und das erhaltene Pellet nachfolgend proteolytisch behandelt und die Nukleinsäure wiederum in an sich bekannter Art über die Anbindung an eine silikatische feste Phase isoliert (QIAamp UltraSens Virus Handbook). Auch hierbei wird auf Probleme mit der Resuspendierung des Pellets hingewiesen. Darüber hinaus erlaubt das Verfahren auch nur die Bearbeitung von Proben mit einem Volumen von maximal 1 ml.

Inhalt der Offenlegungsschrift WO 03/095079 A2 ist eine Filtermembran auf der Basis von auspolymerisierten Alginatfasern sowie weiteren Support-Materialien zum Anreichern/und oder Abtrennen von Bestandteilen aus Flüssigkeiten. Bei den Bestandteilen, die angereichert werden sollen, handelt es sich um Protozoen. Die Anwendung richtet sich auf das Gebiet der Bestimmung und Anreicherung von mikrobiologischen Komponenten im Trinkwasser etc.. Beschrieben wird darin ein Verfahren zur Herstellung einer speziellen Membranschicht (Vlies) auf der Basis eines Polyuronsäurepolymerisats. In einem aufwendigen Prozess wird eine Filtermembran erzeugt, welche dann zweckbestimmt eingesetzt wird. Beim genannten Verfahren erfolgt ein Passagieren einer Flüssigkeit über eine zuvor bereits hergestellte Filtermembran auf der Basis eines Polyuronsäurepolymerisats. Die genannte Offenlegungsschrift gibt keinen Hinweis darauf, das Polymerisat aufzulösen, um eine nachfolgende Isolierung von Nukleinsäuren aus der biologischen Probe zu realisieren oder nach Anreicherung von Viren aus diesen eine Nukleinsäure zu isolieren. Auch gibt es keinen Hinweis darauf, dass mit der Membran Viren angereichert werden können, da dass Anreicherungsprinzip darauf begründet wird, das die Anreicherung der Protozoen in den definiert ausgebildeten Poren des Polyuronsäurepolymerisats erfolgt. Eine Isolierung von Nukleinsäuren aus biologischen Proben wird in WO 03/095079 A2 weder beschrieben, noch nahegelegt. Es gibt auch keine Hinweise darauf, dass das Auflösen des Polymerisats mit einem chaotropen Puffer erfolgt.

Das Patent US 5739019 A beschreibt die Isolierung von Mikroorganismen aus einer wässrigen Probe. Das Patent beschreibt kein Verfahren, bei dem Biomoleküle in mittels Polyuronsäure basierten Polymerisaten aus einer biologischen Proben aufkonzentriert werden und nachfolgend die Isolierung von Nukleinsäuren erfolgt, sondern offenbart den Mechanismus, das man Polyuronsäure durch Zugabe von Kalziumchlorid polymerisieren lässt. In dem zitierten Patent nutzt man diesem Mechanismus, in dem wässrige Lösung aus Kalziumchlorid und Mikroorganismen mit einer Polyuronsäure tröpfchenweise versetzt wird und in diese Tröpfchen dann die Mikroorganismen eingekapselt werden. Diese eingekapselten Mikroorganismen werden dann in nachfolgenden Verfahrenschritten in sog. selektiven Wachstumsmedien kultiviert. Es geht nicht um eine nachfolgende Isolierung von Nukleinsäuren aus den Biomolekülen für einen diagnostischen Nachweis. Ein Hinweis auf die Isolierung von DNA gibt es nicht.

In Tribune du Cebedeau, 1976, 29 (390), S. 186-94 wird über die Isolierung eines Virusses berichtet. Die Isolierung einer Nukleinsäure wird nicht erwähnt. Mittels Alginat und Kalziumchlorid wird in bekannter Form eine Membran erzeugt. Über diesen Membranfilter (polymerisiertes Alginatfiltersystem) erfolgt dann die Passage einer Lösung, aus der Viren an den Filter oder in den Filter adsorbieren sollen. Damit nutzt man das Verfahren, Viren an oder in Membranen (oder besser Hydrogelen) anzureichern, indem man die die Viren enthaltene Flüssigkeit über diese Alginatmembran filtriert.

Die beschriebenen Verfahren nach dem Stand der Technik zeigen deutlich, dass die Bearbeitung von großvolumigen Proben an sich, als auch die Erhöhung des Volumens einer Probe zur Erhöhung der Sensitivität bei Nachweisen viraler Nukleinsäuren, nach wie vor kompliziert und mit erheblichen Nachteilen verbunden ist.

Es ist bisher im Stand der Technik nicht beschrieben worden, dass man Nukleinsäuren an oder in polymerisiertes Alginat binden kann und nachfolgend wiedergewinnen kann. Es ist auch bisher nicht bekannt, dass man Mikroorganismen (speziell Viren) anreichert, um dann nachfolgend daraus Nukleinsäuren zu isolieren, um die Viren nach Aufkonzentrierung nachzuweisen.

Der Erfindung lag deshalb die Aufgabe zu Grunde, eine Anreicherung von Nukleinsäuren oder Viren zu ermöglichen, um insbesondere großvolumige Proben bearbeiten zu können. Das Verfahren soll dabei zu einer sich anschließenden einfachen und schnellen Methode der Nukleinsäureisolierung kompatibel sein.

Die Aufgabe wurde gemäß den Merkmalen der Patentansprüche gelöst.

Überraschenderweise wurde gefunden, dass sich bestimmte Polysaccharid-Derivate hervorragend für eine Anreicherung von Viren in flüssigen Proben einsetzen lassen, wobei dieser Anreicherungsschritt kompatibel zu effizienten Verfahren der Isolierung von viralen Nukleinsäuren ist. Bei den Polysaccharid-Derivaten handelt es sich um die Salze von Polyuronsäuren. Besonders geeignet sind dabei die sog. Alginate der Alginsäuren. Bei Alginaten handelt es sich um strukturgebende Elemente bei Braunalgen. Alginat ist ein Polysaccharid, welches aus 1,4 verknüpfter α-L-Guluronsäure (G) und β-D-Mannuronsäure (M) besteht.

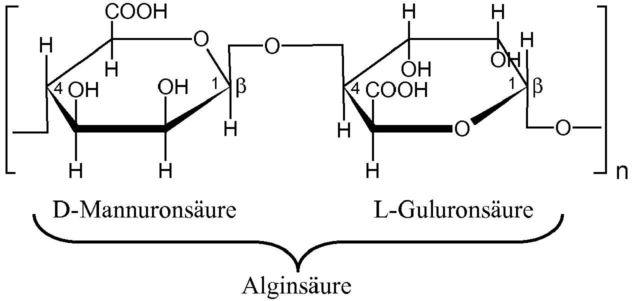

Es bildet homopolymere Bereiche, in denen Mannuronsäure oder Guluronsäure als Block vorliegt.

Alginate werden in der Lebensmittel- sowie der Pharma- und Kosmetikindustrie eingesetzt, z.B. als Geliermittel in der Lebensmittelindustrie, als Apreturmittel für Textilien, zur Herstellung von photographischen Papieren oder in der zahnmedizinischen Praxis zur Herstellung von Zahn- und Kieferabformungen.

Alginat ist auch ein wichtiges Biomaterial. Durch die Verkapselung menschlichen Zellgewebes mit Alginat ist es möglich, körperfremdes Material wie zum Beispiel Spenderzellen einzulagern, ohne dass diese vom Immunsystem erkannt und zerstört werden können. Es gibt jedoch keinerlei Hinweise darauf, das Alginate als Materialien zur Anreicherung von Viren für eine nachfolgende Isolierung und Aufreinigung von viraler Nukleinsäuren eingesetzt werden können.

Für das erfindungsgemäße Verfahren nutzt man die bekannte Fähigkeit von Alginaten, in Lösungen mit niedrigem Kalziumgehalt zu gelieren und sog. Hydrogele zu bilden. Die Ursache der Gelierung begründet sich in der Einlagerung von Kalziumionen in die Zickzackstruktur der GG- Blöcke. Auf diese Zone lagert sich dann die Zickzackstruktur eines anderen Alginatmoleküls. Es kommt hierdurch zur Ausbildung dreidimensionaler Strukturen. Die Ausbildung von Gelen erfolgt auch in Kombination mit starken Säuren. Die entstandenen Gelstrukturen können darüber hinaus auch wieder spezifisch zerstört werden.

Unter Ausnutzung der Ausbildung von Alginatgelen kann die Anreicherung von Viren für eine nachfolgende molekulardiagnostische Analyse aus einer flüssigen Probe, insbesondere aus den problematischen großvolumigen Proben, mittels des erfindungsgemäßen Verfahrens extrem einfach und schnell realisiert werden. Das Verfahren ist dabei hinsichtlich Chemikalieneinsatz ungefährlich und benötig keinerlei Spezialausstattung.

Das erfindungsgemäße Verfahren zur Anreicherung von Viren aus einer Probe für eine nachfolgende Isolierung der viralen Nukleinsäuren beinhaltet folgende Schritte:
1. Zugabe einer wässrigen Lösung eines Salzes einer Polyuronsäure zur Probe
2. Zugabe einer Substanz, welche die Gelausbildung/ Pelletbildung induziert (z.B. Verwendung einer 1 M Kalziumchloridlösung oder einer 1%igen Salzsäurelösung)
3. Mischen der Probe und Inkubation bei Raumtemperatur
4. Zentrifugation der Probe und Entfernung des Überstandes
5. Auflösen des Pellets oder Gelstückchens und nachfolgende Isolierung der viralen Nukleinsäuren in an sich bekannter Art und Weise

Im Unterschied zum Stand der Technik wird keine auspolymerisierte Membran eingesetzt, sondern zwei Komponenten die - in einen direkten Kontakt gebracht - ein Gel erzeugen können, wobei diese Komponenten in Kontakt mit einer Probe gebracht werden, aus der Nukleinsäuren und oder andere Biomoleküle isoliert werden. Das Gelstück mit den enthaltenen angereicherten Biomolekülen der Probe wird dabei erst sichtbar nach einer Zentrifugation. Vorher ist die Probe nach wie vor flüssig und nicht auspolymerisiert.

Im Unterschied zum Stand der Technik wird kein Verfahren benutzt, bei dem sich kultivierte Mikroorganismen in einer wässrigen Lösung befinden, die bereits eine komplexierende Komponente nach dem enthält (z.B. wässrige Lösung aus Kalziumchlorid) und man dann durch tröpfchenweise Zugabe einer Alginatlösung quasi auspolymerisierte Tröpfchen erhält, die dann die Mikroorganismen eingeschlossen haben.

Das erfindungsgemäße Verfahren unterscheidet sich von dem Verfahren, das in WO 03/095079 A2 beschrieben wurde dadurch, dass keine Membran auf der Basis von polymerisierter Polyuronsäure verwendet wird, sondern basiert auf der Kombination einer Polyuronsäure mit einer biologischen Probe und einer nachfolgenden Komponente, die eine Gelbildung ermöglicht. Dabei bleibt die biologische Probe bestehen, das Polymerisat entsteht erst nach erfolgter Zentrifugation, da nicht die gesamte Probe auspolymerisiert. Damit grenzt sich das erfindungsgemäße Verfahren deutlich von WO 03/095079 A2 ab. Auch ist es Ziel des erfindungsgemäßen Verfahrens, das Polymerisat aufzulösen, um eine nachfolgende Isolierung von Nukleinsäuren aus der biologischen Probe zu realisieren (auch nach Anreicherung von Viren aus diesen eine Nukleinsäure zu isolieren).

Die im erfindungsgemäßen Verfahren eingesetzten Konzentrationen an Alginat und Reagenz zur Gelausbildung sind extrem gering. Daraus folgt, dass der Prozess der Gelierung sichtbar gar nicht erfolgt, wie dies aus der Lebensmittelindustrie bekannt ist oder wie dies für alle Anwendung von Alginaten beschrieben ist. Nach Zentrifugation wird der Überstand entfernt. Es wird ein kleines Gelstück oder Pellet am Boden des Reaktionsgefäßes sichtbar, dieses Gelstück/ Pellet enthält die aus der Probe aufkonzentrierten Viren. Nachfolgend wird das Gelstück/ Pellet durch Zugabe einer Lösung, welche die Gelstruktur zerstört, wieder aufgelöst. Im letzten Schritt erfolgt dann die Isolierung der viralen Nukleinsäuren aus der aufkonzentrierten Probe mit bekannten Methoden der Isolierung von Nukleinsäuren. Das Verfahren ist extrem einfach in der Durchführung. Die Inkubationszeit und nachfolgende Zentrifugation kann innerhalb von 5 - 10 min abgeschlossen sein. Die Auflösung des Gelstücks erfolgt problemlos. Die bekannten Probleme mit einer extrem schweren Resuspendierbarkeit von PEG/ NaCl- Präzipitaten treten nicht auf. Es wird keine Ultrazentrifugation benötigt, so das mit normalen Standardtischzentrifugen gearbeitet werden. Die Volumen der flüssigen Ausgangsproben können beliebig gewählt werden, was ein sehr breites Anwendungsspektrum erlaubt. Es kann z.B. mit Proben von 500 µl oder aber auch 10 ml gearbeitet werden. Damit ist eine enorm große Applikationsbreite gegeben.

Durch das erfindungsgemäße Verfahren reduziert sich das initiale Probenvolumen auf das im erfindungsgemäßen Prozess entstandenen Gelstück/ Pellet, welches dann problemlos im sog. Mini- Format der Nukleinsäureisolierung weiterbearbeitet werden kann.

Für das erfindungsgemäße Verfahren kombiniert man z.B. eine wässrige Alginatlösung und eine wässrige Lösung, enthaltend Salze von di- bzw. polyvalenten Kationen (z.B. Kalziumchlorid oder Aluminiumchlorid). Ebenso kann man eine wässrige Alginatlösung und eine schwache Säure (Salzsäure) kombinieren. Zerstörung der Gelstruktur kann mittels einer Pufferlösung, welche einen Chelatbilder (EDTA) enthält oder aber auch mittels der Zugabe einer Lösung aus tri-Natriumcitrat-Dihydrat erfolgen. Ebenso kann man das Gelstück/ Pellet in einem Niedrigsalzpuffer (z.B. 10 mM Tris- HCl) bei einem basischen pH- Wert auflösen.

Eine besonders effiziente Ausführungsform nutzt den beobachteten Effekt, dass die Auflösung der entstandenen Alginatgele auch mit Puffern, welche für die Isolierung von Nukleinsäuren eingesetzt werden, hervorragend möglich sind. Dabei zerstören sog. chaotrope Puffer z.B. auf der Basis von Guanidiniumsalzen Alginatgele in einer generellen Art und Weise unabhängig davon, durch welches Verfahren das Alginatgel ausgebildete wurde. Das Fachliteratur zu Alginaten und deren Anwendungsgebieten führt einen solchen beobachteten Effekt nicht auf.

Basierend auf dieser Beobachtung kann das Alginatgel dann mit einem chaotropen Puffer aufgelöst werden, welcher nachfolgend gleichzeitig für den Prozess der Isolierung der viralen Nukleinsäuren in einer Dualfuktion genutzt werden kann. In diesem bevorzugten Fall wird nach Zentrifugation und Entfernung des Überstandes das Alginatgel mit einem chaotropen Puffer aufgelöst und der Ansatz nachfolgend mit einem mineralischen Trägermaterial in Kontakt gebracht. Optional können dem chaotropen Puffer auch noch weitere Komponenten zugegeben werden (Alkohole oder Detergenzien oder Mischungen von Alkoholen und Detergenzien), die eine Optimierung der Anbindung der viralen Nukleinsäuren an das mineralische Trägermaterial verstärken können. Die gebundenen Nukleinsäuren werden dann gewaschen und final wieder vom Trägermaterial abgelöst. Die Isolierung von viralen Nukleinsäuren über die erfindungsgemäße Virus-Anreicherung aus einer großvolumigen Probe kann dann in der Regel schon innerhalb von ca. 30 min ermöglicht werden. Das erfindungsgemäße Verfahren ist damit deutlich einfacher und schneller als alle anderen diesbezüglichen Verfahren und auch deutlich schneller als bisher bekannte Techniken. Damit löst das erfindungsgemäße Verfahren die beschriebene Problemstellung ideal.

Eine weitere diagnostische Herausforderung betrifft die Isolierung von sog. freien oder nackten Nukleinsäuren aus biologischen Proben. Auch hierbei wäre es wünschenswert, ein großes Volumen der zu untersuchenden Probe (z.B. Urin, Serum, Plasma) einzusetzen, da auf Grund der sehr geringen Konzentrationen an freien Nukleinsäuren die Bearbeitung von kleinen Probenmengen keine umfassende diagnostische Analyse ermöglicht. Eine Erhöhung an Sensitivität kann nur noch über die Erhöhung des Probenvolumens realisiert werden. Dafür existieren aber keine Verfahren in der Laborpraxis.

Überraschenderweise zeigt sich, dass wiederum Salze von Polyuronsäuren, insbesondere Alginate oder Alginsäuren, auch in der Lage sind, Nukleinsäuren aus Proben anzureichern bzw. generell als Material für die Isolierung von Nukleinsäuren in exzellenter Form eingesetzt werden können. Dies ist umso unerwarteter, da es sich bekannterweise bei Nukleinsäuren wie auch bei Alginaten um Polyanionen handelt. Insofern war es schon sehr überraschend, mittels dieser Zucker auch Nukleinsäuren anreichern und nachfolgend isolieren zu können. Im erfindungsgemäßen Verfahren bedient man sich dabei wieder dem Verfahrensablauf, welcher auch für die Anreicherung von Viren aus Proben beschrieben wurde:
1. Zugabe einer wässrigen Lösung eines Salzes einer Polyuronsäure zur Probe
2. Zugabe einer Substanz, welche die Gelausbildung induziert (z.B. Verwendung einer 1 M Kalziumchloridlösung oder einer 1%igen Salzsäurelösung)
3. Mischen der Probe und Inkubation bei Raumtemperatur
4. Zentrifugation der Probe und Entfernung des Überstandes
5. Auflösen des Gelstückchens

Die Isolierung der angereicherten freien Nukleinsäuren kann dann wiederum dadurch erfolgen, dass man das Gelstück/ Pellet mit bereits beschriebenen Puffern auflöst und die Nukleinsäuren dann mit an sich bekanten Methoden z.B. über die Anbindung an mineralische feste Phasen isoliert. Auch kann man wiederum zur Auflösung des Gelstücks/ Pellets chaotrope Puffer einsetzen und nachfolgend die Isolierung der Nukleinsäuren durchführen. Die Aufreinigung kann allerdings auch mit klassischen Verfahren, z.B. über die Fällung der Nukleinsäuren, erfolgen.

Ein besonders einfaches Verfahren der Isolierung freier Nukleinsäuren aus Proben besteht darin, die Probe mit einer wässrigen Alginatlösung und einer Säure zu mischen. Nach Zentrifugation wird der Überstand entfernt und das Gelstück mit einem basischen Niedrigsalzpufffer (z.B. 10 mM Tris-HCl; pH 9) aufgelöst. Die Aufreinigung der Nukleinsäuren ist nicht notwendig. Die Lösung kann sofort für z.B. PCR- Applikationen eingesetzt werden. Hier zeigt sich das Alginate auch eine Verstärkung der Amplifikationsreaktion bewirken können.

Eine solche Aufreinigungsvariante ist in wenigen Minuten abgeschlossen und bedeutet die einfachste Isolierung von Nukleinsäuren überhaupt. Sie umfasst nochmals zusammengefasst die Schritte:
1. Zugabe einer wässrigen Lösung eines Salzes einer Polyuronsäure zur Probe
2. Zugabe einer Säure zur Probe
3. Mischen der Probe und Inkubation bei Raumtemperatur
4. Zentrifugation der Probe und Entfernung des Überstandes
5. Auflösen des Gelstückchens in einem Niedrigsalzpuffer mit leicht alkalischem pH-Wert
6. Verwendung der isolierten Nukleinsäure sofort in einer PCR

Auch bei dieser speziellen Ausführungsform gibt es praktisch keine Limitierung in Bezug auf das Volumen der Probe.

Das erfindungsgemäße Verfahren ist auch geeignet, generell Nukleinsäuren aus einer Probe zu isolieren. Liegen die zu isolierenden Nukleinsäuren nicht als freie Nukleinsäuren vor, so kann die Probe in an sich bekannter Form lysiert werden. Die dadurch aus der Probe freigesetzten Nukleinsäuren werden danach wiederum entsprechend dem erfindungsgemäßen Verfahren isoliert. Auch hierbei kann man nach dem Auflösen des Gelstücks (welches die angereicherten Nukleinsäure enthält) eine "klassische" Aufreinigung der Nukleinsäuren durchführen oder die nach Auflösung des Gelstücks mittels des beschriebenen basischen Niedrigsalzpuffers die erhaltene Lösung direkt für eine PCR- Reaktionen einsetzen.

Neben der beschriebenen Anreicherung von Viren aus unterschiedlichsten Proben, der Anreicherung von "nackten Nukleinsäuren" als auch der Anreicherung von nicht "freien" Nukleinsäuren werden unspezifisch auch andere Biomoleküle wie Proteine, Enzyme, Antikörper oder Zellrezeptoren einer Probe mittels der beschriebenen Verfahren angereichert. Diese können dann nach dem Fachmann bekannter Art und Weise ebenso wie die Nukleinsäuren für weitere downstream- Anwendungen eingesetzt werden.

Damit stellt das hier offenbarte Verfahren eine neuartige und universelle Möglichkeit der Anreicherung von unterschiedlichsten Biomolekülen für eine spezifische Bearbeitung dar. Es ist extrem schnell und einfach in der Durchführung und bedarf auch keinerlei spezieller Geräte.

Die Universalität der Erfindung wird nachfolgend anhand von Ausführungsbeispielen näher erläutert, wobei die Ausführungsbeispiele keine Limitation des erfindungsgemäßen Verfahrens bedeuten.

### Ausführungsbeispiele

### Ausführungsbeispiel 1: Anreicherung von Nukleinsäuren in einer lysierten biologischen Probe und nachfolgender direkte Einsatz der wieder freigesetzten Nukleinsäuren für eine PCR

Die Anreicherung und nachfolgende Isolierung der Nukleinsäuren erfolgt aus einer Tupferprobe (Abstrich der Mundschleimhaut) und wird wie folgt durchgeführt:
1. Tupfer in ein 1.5 ml Reaktionsgefäß stecken. Zugabe von 400 µl Lysepuffer (5% Triton X-100, 0.1 M Guanidinhydrochlorid) und 10 µl Proteinase K (20mg/ml). Inkubation bei 50°C für 5 min. Tupfer ausdrücken und verwerfen.
2. Zugabe von 10 µl einer wässrigen Alginat- Lösung (1%) sowie von 50 µl 1%iger Salzsäure. Vortexen und bei RT für 2 min inkubieren.
3. Zentrifugation bei Maximalgeschwindigkeit für 1 min. Überstand vollständig entfernen.
4. Zugabe von 1 ml Wasser und Zentrifugation bei Maximalgeschwindigkeit für 1 min. Überstand vollständig entfernen.
5. Zugabe von 200 µl eines Niedrigsalzpuffers (10mM Tris HCl, pH 9). Gelstück/ Pellet resuspendieren und nachfolgend vollständig auflösen.

Die isolierte DNA wurde nachfolgend für eine PCR zur Amplifikation einer humanspezifischen Nukleinsäuresequenz eingesetzt. Das Ergebnis zeigt die Eignung der Nukleinsäure für eine PCR Anwendung.

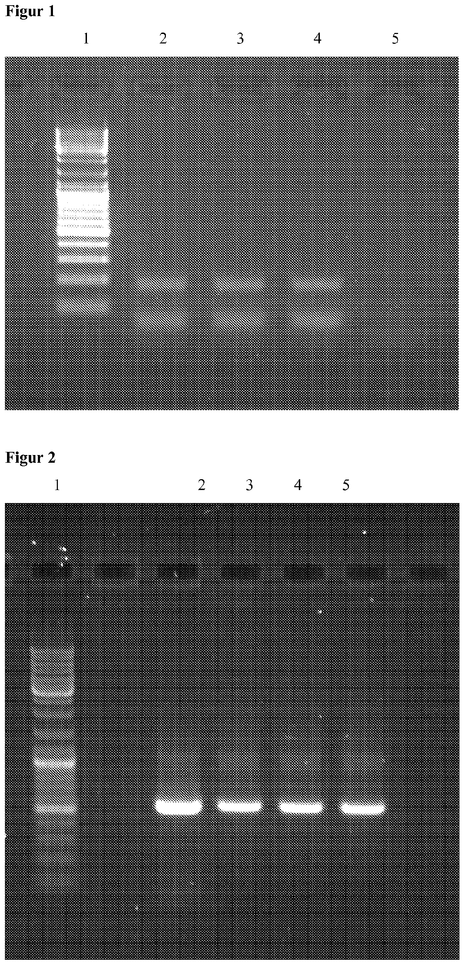

Figur 1 zeigt den Nachweis des Amplifikationsproduktes auf einem Agarosegel.

Die Bezugszeichen in Figur 1 bedeuten:
1- DNA Längenstandard
2- 4 Amplifikationsprodukte
5 - PCR Leerwert

### Ausführungsbeispiel 2: Anreicherung von freien Nukleinsäuren ("nackte" Nukleinsäure) aus einer großvolumigen Probe in Kombination mit einer nachfolgenden Nukleinsäureaufreinigung

In 5 ml einer Wasserprobe (Dreifachbestimmung) wurde ein DNA- Fragment von 560 bp gespiked. Die Probe wird in ein 15 ml Zentrifugationsgefäß überführt. Die Isolierung der freien Nukleinsäure wird wie folgt durchgeführt:
1. Zugabe von 100 µl einer wässrigen Alginat- Lösung (1%) sowie von 500 µl 1%igen Salzsäure. Vortexen und bei RT für 5 min inkubieren.
2. Zentrifugation bei 4500 rpm für 10 min. Überstand vollständig entfernen.
3. Resuspendieren des Gelstücks mit 500 µl einer chaotropen Salzlösung (4 M Guanidinisothyocyanat)
4. Überführen des Ansatzes auf eine Spin Filter Säule mit Glasfasermaterial (Whatmann GFD). Zentrifugation bei 12.000 rpm für 1 min. Verwerfen des Filtrates.
5. Waschen der Spin Filter Säule mit einem ethanolhaltigen Waschpuffer (80% Ethanol). Zentrifugation bei 12.000 rpm für 1 min. Verwerfen des Filtrates.
6. Trocknung des Spin Filter Säule durch kurze Zentrifugation.
7. Spin Filter Säule in ein neues Reaktionsgefäß stecken. Zugabe von 100 µl eines Niedrigsalzpuffers (10 mm Tris HCl) und Elution der Nukleinsäure von der Säule.

Die isolierte freie Nukleinsäure wurde nachfolgend auf einem Agarosegel aufgetragen. Wie zu ersehen, konnte eine nahezu quantitative Rückgewinnung mit dem Verfahren erreicht werden.

Figur 2 zeigt die gelelektrophoretische Darstellung des zurückgewonnenen DNA-Fragmentes.

Die Bezugszeichen in Figur 1 bedeuten:
1- DNA Längenstandard
2- DNA Fragment vor der Aufreinigung
3- 5 DNA Fragmente nach der Aufreinigung aus 5ml Wasser

### Ausführungsbeispiel 3: Virus- Anreicherung und nachfolgende Isolierung der viralen Nukleinsäuren. Beweis der deutlichen Erhöhung der Sensitivität durch den Einsatz einer größeren Probenmenge.

Einem Zellkulturmedium wurden in verschiedenen Konzentrationen Serum mit enthaltenen BVDV- Viren zugesetzt. Für die Anreicherung der Viren wurden 1,8 ml der Probe eingesetzt. Im Vergleich zur Anreicherungsmethode wurde mittels eines Standardverfahrens die virale Nukleinsäure aus 200 µl der Probe isoliert. Der Nachweis der BVDV- Viren erfolgte mit einem kommerziellen Realtime- Testsystem. Es wurden je Verdünnung zwei Proben analysiert. Die in der Tabelle aufgeführten CT-Werte sind die Mittelwerte aus den beiden Proben. Die Anreicherung der Viren wurde wie folgt durchgeführt.
1. Überführen von 1,8 ml der virusenthaltenen Zellkulturprobe in ein 2,0 ml Reaktionsgefäß. Zugabe von 25 µl einer wässrigen Alginat- Lösung (1%) sowie von 200 µl 1%igen Salzsäure zum Zellkulturmedium (2ml). Vortexen und bei RT für 10 min inkubieren.
2. Zentrifugation bei 14.000 rpm für 2 min. Überstand vollständig entfernen.
3. Resuspendieren des Gelstücks mit 500 µl einer chaotropen Salzlösung (4 M Guanidinisothyocyanat)

Die nachfolgende Isolierung der viralen Nukleinsäuren erfolgte auf einem Extraktionsautomaten (InnuPURE C12; Analytik Jena AG). Dabei erfolgt mittels eines kommerziellen Kits (innuPREP Virus RNA Kit; Analytik Jena AG).

Die direkte Aufarbeitung von 200µl Proben erfolgte ebenfalls mit dem kommerziellen Kit auf einem Extraktionsautomaten (InnuPURE C12; innuPREP RNA Kit C12; analytik Jena AG)

Nachfolgende wurde die isolierte Nukleinsäure für den Virusnachweis mittels Realtime- PCR eingesetzt.

Die Ergebnisse der Realtime- PCR sind nachfolgend aufgeführt.

**Ergebnisse der Real Time PCR - Vergleich der "Standardmethode" mit der erfindungsgemäßen Anreicherungsmethode**

| | "direktes Verfahren unter Verwendung von 200 µl Probe | Anreicherungsverfahren unter Verwendung von 1.8 ml Probe |
|---|---|---|
| Verdünnung 1 | CT 32,8 | CT 31,4 |
| | | |
| Verdünnung 2 | CT 36,2 | CT 33,5 |
| | | |
| Verdünnung 3 | CT 39,7 | CT 37,7 |

Damit wird deutlich, das mit dem erfindungsgemäßen Anreicherungsverfahren eine größere Probenmenge bearbeitet werden kann. Daraus resultiert der Vorteil einer höheren Sensitivität, insbesondere wenn die Anzahl nachzuweisender Viren gering ist. Damit zeigt sich, dass das erfindungsgemäße Verfahren ein Möglichkeit bietet größere Probenvolumina zu bearbeiten und damit eine höhere Nachweisempfindlichkeit zu erreichen.

## Patentansprüche

1. Verfahren zur Anreicherung und Isolierung von Nukleinsäuren und/oder Viren aus einer Probe, **gekennzeichnet durch** folgende Schritte:
a) Zugabe einer wässrigen Lösung eines Salzes einer Polyuronsäure zur Probe
b) Zugabe einer Substanz, welche die Gelbildung/ Pelletbildung der Polyuronsäure induziert
c) Mischen der Probe und Inkubation
d) Zentrifugation der Probe und Entfernung des Überstandes
e) Auflösen des Pellets oder Gelstückchens
f) Isolierung der Nukleinsäuren und/oder viralen Nukleinsäure in an sich bekannter Art und Weise.

2. Verfahren nach Anspruch 1, **dadurch gekennzeichnet, dass** als Salze einer Polyuronsäure Alginate eingesetzt werden.

3. Verfahren nach einem der Ansprüche 1 oder 2, **dadurch gekennzeichnet, dass** als Substanz, welche die Gelbildung/ Pelletbildung der Polyuronsäure induzieren Säuren und / oder Lösungen, die Kalziumionen enthalten, verwendet werden.

4. Verfahren nach Anspruch 3, **dadurch gekennzeichnet, dass** die Konzentration der Kalziumionen-Lösung 1mol/l beträgt

5. Verfahren nach Anspruch 3, **dadurch gekennzeichnet, dass** als Säure eine 1%ige Säure verwendet wird.

6. Verfahren nach einem der Ansprüche 1 bis 5, **dadurch gekennzeichnet, dass** zum Auflösen des Pellets oder Gelstückchens Alkalien, Chelatbilder oder chaotrope Salze eingesetzt werden.

7. Verfahren nach Anspruch 6, **dadurch gekennzeichnet, dass** EDTA, Guanidiniumsalze oder Citrate eingesetzt werden.

8. Verfahren nach einem der Ansprüche 1 bis 7, **dadurch gekennzeichnet, dass** vor Beginn des Verfahrens oder nach Abschluss des Verfahren eine Lyse der Probe durchgeführt wird.

## Claims

1. A method for concentrating and isolating nucleic acids and / or viruses from a sample **characterized by** the following steps:
a) adding an aqueous solution of a salt of a polyuronic acid to the sample
b) adding a substance which induces gel formation / pellet formation of the polyuronic acid
c) mixing the sample and incubation
d) subjecting the sample to centrifugation and removing the supernatant
e) dissolving the pellet or gel piece
f) isolating the nucleic acids and / or viral nucleic acid in a known fashion.

2. The method according to claim 1, **characterized in that** alginates are used as salts of a polyuronic acid.

3. The method according to claim 1 or 2, **characterized in that** as a substance which induces gel formation / pellet formation of the polyuronic acid acids and / or solutions are used which contain calcium ions.

4. The method according to claim 3, **characterized in that** the concentration of the calcium ion solution is 1 mol/l.

5. The method according to claim 3, **characterized in that** a 1 % acid is used as an acid.

6. The method according to any one of claims 1 to 5, **characterized in that** for dissolving the pellet or gel piece alkalis, chelating agents or chaotropic salts are used.

7. The method according to claim 6, **characterized in that** EDTA, guanidinium salts or citrates are used.

8. The method according to any one of claims 1 to 7, **characterized in that** prior to start of the method or after completion of the method a lysis of the sample is carried out.

## Revendications

1. Procédé pour la concentration et l'isolation des acides nucléiques et / ou des virus à partir d'un échantillon, **caractérisé par** les étapes suivantes:
a) ajouter une solution aqueuse d'un sel d'un acide polyuronique à l'échantillon
b) ajouter une substance induisant la formation d'un gel / la formation d'un pellet de l'acide polyuronique
c) mélanger l'échantillon et incubation
d) centrifuger l'échantillon et enlever le surnageant
e) dissoudre le pellet ou le morceau de gel
f) isoler les acides nucléiques et / ou l'acide nucléique viral d'une manière connue.

2. Procédé selon la revendication 1, **caractérisé en ce que** des alginates sont utilisés en tant que sels d'un acide polyuronique.

3. Procédé selon l'une quelconque des revendications 1 ou 2, **caractérisé en ce que** des acides et / ou des solutions contenant des ions calcium sont utilisés en tant que substance induisant la formation d'un gel / la formation d'un pellet de l'acide polyuronique.

4. Procédé selon la revendication 3, **caractérisé en ce que** la concentration de la solution des ions calcium est 1 mol/l.

5. Procédé selon la revendication 3, **caractérisé en ce qu'** un acide de 1 % est utilisé en tant que l'acide.

6. Procédé selon l'une quelconque des revendications 1 à 5, **caractérisé en ce que** pour dissoudre le pellet ou le morceau de gel des alcalis, des agents de chélation ou des sels chaotropes sont utilisés.

7. Procédé selon la revendication 6, **caractérisé en ce qu'**EDTA, des sels de guanidinium ou des citrates sont utilisés.

8. Procédé selon l'une quelconque des revendications 1 à 7, **caractérisé en ce qu'**avant le début du procédé ou après la fin du procédé une lyse de l'échantillon est effectuée.
